Europäisches Patentamt

European Patent Office

Office européen des brevets

(11) Publication number: **0 259 034 A2**

(12) **EUROPEAN PATENT APPLICATION**

(21) Application number: 87307147.6

(22) Date of filing: **12.08.87**

(51) Int. Cl.⁴: **A61K 7/00** , G01N 1/00

(30) Priority: **27.08.86 US 900749**

(43) Date of publication of application:
**09.03.88 Bulletin 88/10**

(84) Designated Contracting States:
**CH DE ES FR GB IT LI**

(71) Applicant: **MINNESOTA MINING AND MANUFACTURING COMPANY**
**3M Center, P.O. Box 33427**
**St. Paul, Minnesota 55133-3427(US)**

(72) Inventor: **Garbe, James E. c/o Minnesota Mining and**
**Manufacturing Company 2501 Hudson Road**
**St. Paul Minnesota 55133-3427(US)**

(74) Representative: **Baillie, Iain Cameron et al**
**c/o Ladas & Parry Isartorplatz 5**
**D-8000 München 2(DE)**

(54) **Cosmetic sampling device.**

(57) Wax-base or emollient-base cosmetic composi-
tions may be provided as samples on paper carrying
sheets by providing the paper with materials that
prevent absorption of oleic materials and providing
the cosmetic compositions with a lower sheet that is
oleophobic.

EP 0 259 034 A2

## COSMETIC SAMPLING DEVICE

### BACKGROUND OF THE INVENTION

#### 1. Field of the Invention

Cosmetic compositions having a wax or emollient base are provided in a structure suitable for sampling which is a multilayered article comprising a paper carrying sheet using both a) chemical impregnants which prevent absorption of oleic materials and b) an oleophilic surface in contact with the surface of the cosmetic composition.

#### 2. Background of the Art

Cosmetic samples of wax-or emollient-base compositions are often provided to the potential consuming public in conventional commercial forms. Lipstick tubes are available for general use at the sales counter as are bottles of foundation and other cosmetics. The open and general use of these samples by the public at large offers some potential health risks which should be avoided.

### SUMMARY OF THE INVENTION

Samples of solid cosmetic compositions having a wax or emollient carrying medium can be provided on paper carrying sheets. The paper is impregnated or coated with a material that prevents absorption of oleic materials and a sheet or film with an oleophilic surface in contact with the exposed surface of the cosmetic composition.

### DETAILED DESCRIPTION OF THE INVENTION

Wax-base and emollient-base cosmetic compositions are not stable when coated in paper bases and therefore can not readily be sampled in that manner. The hydrocarbon, oleic, or lower molecular oleophilic materials seep into the paper and leave behind a cosmetic composition with less than optimal properties. Additionally, the appearance of oil stains around the cosmetic composition from that seepage is less than attractive to the potential buyer. Commercially available papers which are treated with polymeric and particulate compositions to prevent oil penetration have not been able to prevent penetration by these cosmetic compositions.

The use of penetrating compositions which are oleophobic has proved to be very useful in the solution of one aspect of this problem. These compositions should penetrate into the surface and preferably all the way through the paper to provide oleophobic properties. To be considered oleophobic, the treated paper should be able to be melt coated with a 0.1 mm thick 4 cm² patch of carnauba wax with a dye dissolved therein so that after two weeks at 30°C and 40% relative humidity, there is not more than a 15% gain in surface area with visible oil stain or coloration thereon. Preferably there is no more than a 5% gain in stained or colored surface area.

Cosmetic samples are usually provided in either single folded or overlaid sheets of as an insert or page in a magazine or brochure. The oleophobic surface or layer which is in contact with the solid cosmetic composition layer can be loosely (i.e., strippably) adhered to the surface of the composition, overlaid on the cosmetic material (held in place by a face of the folded sheet or magazine page), or adhered to a facing (opposite) page. The oleophilic layer may be easily strippably adhered to the cosmetic material. If the cosmetic composition is applied as a hot melt or dryable composition, the oleophilic layer may be laid on top contemporaneously with that application. The layer may also be placed on top and heated, or used with a moderate-or low-strength pressure-sensitive adhesive, after application of the layer to the paper base.

The oleophobic penetrating composition can be selected from a number of commercially available types of materials such as fluorocarbons (e.g., Scotchban™, a salt of a highly fluorinated polymer or a fluorochemical phosphate ester) and fluorochemical phosphates. Other materials such as fluorinated polymers and compositions such as those described in U.S. Patent No. 4,529,658 are also useful. The disclosure of that patent is incorporated herein by reference with respect to the compositions and disclosure of chemical materials described therein. The preferred composition is a fluorochemical polymer, particularly those derived from about 60 to 80% of fluorochemical acrylates, 1 to 30% alkyl or alkoxyalkyl acrylates (inclusive also of methacrylates), 2 to 15% glycidyl acrylate (inclusive also of methacrylates), 1 to 6% cationic acrylates (inclusive also of methacrylates) and 0 to 20% vinylidene chloride. These materials are used in amounts of 0.002 to 3% by weight of paper in the base, preferably 0.02 to 2% by weight of said paper. Other fluorochemical polymers are used in the same weight ratios.

The oleophobic surface may be a film, laminated film, coated surface, foil, metallized surface, and the like. Many polymeric materials are useful for the film including but not limited to acrylates, polyamides, polyvinyl resins/polyvinyl alcohol, polyvinylidene chloride, polyvinyl chloride, silicone resins, and the like.

The cosmetic compositions are limited to those containing at least 5% by weight of an emollient or wax material. Compositions with lower contents of these oleic materials do not need to practice the present invention. Other conventional cosmetic additives may of course be present in the cosmetic layer. Such materials as polymeric binders, oils, water, dyes, pigments, moisturizers, fragrances, filler, antioxidants, emulsifying agents, and the like are often present.

The cosmetic composition is preferably applied to the sampler in sufficient quantity so that it may be transferred to a person's skin in amounts that will approximate normal usage of the cosmetic. Coating thicknesses of at least 10 microns and more preferably at least 50 microns are desirable. Coating thicknesses in the range of 0.05 to 2.0 mm and preferably 0.1 to 1.0 mm are generally used.

Stacks of sampler sheets are strippably adhered together at the edges or through the cosmetic material as an adhesive. This last construction can be effected by having the backside of the upper samples, in contact with the cosmetic coating layer, coated with or laminated to the oleophobic layer.

These and other aspects of the present invention will be described in the following non-limiting examples.

Example 1

Two sheets of two-side coated printing stock were used in this example. The paper is coated on both sides with a clay and polymer coating composition. The paper was treated with 1% by weight of the paper of Scotchban™ oil repellent, an oligomeric fluorinated ester. Both the coating and the paper itself absorbed the oil repellent. A stick of commercially available wax base lipstick was gently melted and hand coated onto one surface of the paper base. A polyvinylidene chloride film (7.6 x $10^{-5}$ m) was pressed onto the cosmetic coating while it was still warm. This procedure was duplicated with untreated paper and the two samples stored for two weeks at 20-25°C.

The sample on the untreated paper showed oil stain in the paper surrounding the cosmetic coating. No stain could be seen in the sample with the treated sample.

Claims

1. A multilayered structure for sampling cosmetic compositions comprising a paper base having an oleophobic agent within said paper, a solid cosmetic composition layer containing a wax or emollient component on at least a portion of one surface of said paper base and an oleophobic surface in contact with said solid cosmetic composition layer.

2. The structure of claim 1 wherein said oleophobic agent is throughout the paper base.

3. The structure of claim 1 wherein said paper base comprises paper coated with pigment and binder.

4. The structure of claim 3 wherein said oleophobic agent is present within said paper and said coating of pigment and binder.

5. The structure of claims 1-4 wherein said oleophobic agent is a fluorocarbon.

6. The structure of claims 1-4 wherein said oleophobic surface is a polymeric film strippably adhered to the surface of the cosmetic composition layer.

7. The structure of claim 5 wherein said oleophobic surface is a polymeric film strippably adhered to the surface of the cosmetic composition layer.

8. The structure of claims 1-4 wherein said oleophobic surface is a paper base with an oleophobic polymeric composition thereon comprising a fluorinated polymer or phosphate ester of a fluorinated polymer.

9. The structure of claims 1-4 wherein said oleophobic surface is a paper base with an oleophobic polymeric composition thereon comprising by weight
60 to 80% fluorochemical acrylate,
1 to 30% alkyl-or alkoxyalkyl-acrylate,
2 to 15% glycidyl acrylate,
1 to 6% cationic acrylate, and
0 to 20% vinylidene chloride.

10. The structure of claims 1-4 wherein said cosmetic composition comprises lipstick.